(19) Europäisches Patentamt
European Patent Office
Office européen des brevets

(11) **EP 3 129 081 B1**

(12) **EUROPÄISCHE PATENTSCHRIFT**

(45) Veröffentlichungstag und Bekanntmachung des Hinweises auf die Patenterteilung:
**10.08.2022   Patentblatt 2022/32**

(21) Anmeldenummer: **15722651.5**

(22) Anmeldetag: **31.03.2015**

(51) Internationale Patentklassifikation (IPC):
***A61M 1/28*** *(2006.01)*

(52) Gemeinsame Patentklassifikation (CPC):
**A61M 1/282; A61M 1/28; A61M 1/285;** A61M 2205/3334

(86) Internationale Anmeldenummer:
**PCT/EP2015/000698**

(87) Internationale Veröffentlichungsnummer:
**WO 2015/154859 (15.10.2015 Gazette 2015/41)**

(54) **DIALYSEGERÄT ZUR ABSCHÄTZUNG DER BEHANDLUNGSDAUER EINER PERITONEALDIALYSE-BEHANDLUNG**

DIALYSIS DEVICE FOR ESTIMATING THE DURATION OF A PERITONEAL DIALYSIS TREATMENT

DISPOSITIF DE DIALYSE POUR L'ESTIMATION DE LA DURÉE D'UN TRAITEMENT DE DIALYSE PÉRITONÉALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.04.2014   DE 102014005122**

(43) Veröffentlichungstag der Anmeldung:
**15.02.2017   Patentblatt 2017/07**

(73) Patentinhaber: **Fresenius Medical Care Deutschland GmbH**
**61352 Bad Homburg (DE)**

(72) Erfinder:
• **GRIESSMANN, Erik**
**97422 Schweinfurt (DE)**
• **BUDA, Martin**
**97456 Dittelbrunn (DE)**
• **WOLF, Klaus**
**97450 Arnstein (DE)**

(74) Vertreter: **Herrmann, Uwe**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
**US-A1- 2010 004 589     US-A1- 2012 029 937**
**US-A1- 2012 071 815     US-A1- 2013 006 171**

**Beschreibung**

Die vorliegende Erfindung betrifft eine Vorrichtung, die dazu ausgelegt ist, ein Verfahren zur Abschätzung der Behandlungsdauer einer Peritonealdialyse-Behandlung durchzuführen.

Im Rahmen einer Peritonealdialyse-Behandlung wird einem Patienten zur Entfernung von Schadstoffen aus dem Blut eine Dialyselösung in die Bauchhöhle eingeführt. Dazu dient ein operativ eingesetzter Katheter, mittels dessen die sterile Dialyselösung eingeleitet wird. Diese Dialyselösung nimmt über das als semipermeable Membran dienende Bauchfell, d.h. über das Peritoneum des Patienten Schadstoffe aus dem Blut des Patienten auf. Zusätzlich wird dem Patienten aufgrund des in der Dialyselösung enthaltenen Traubenzuckers und des daraus resultierenden osmotischen Drucks Wasser entzogen. Nach einer bestimmten Verweildauer in der Bauchhöhle wird die Lösung über den Katheter wieder abgelassen und durch eine frische Dialyselösung ersetzt.

Aus dem Stand der Technik sind unterschiedliche Peritonealdialyse-Verfahren bekannt, wie beispielsweise die kontinuierliche ambulante Peritonealdialyse (CAPD), bei der die Patienten selbst ca. vier bis fünf mal pro Tag mit Hilfe eines Beutelsystems die Dialyselösung wechseln. Bei der automatischen Peritonealdialyse übernimmt dies ein Dialysegerät, der sog. Cycler, so dass auch eine Behandlung über Nacht möglich ist.

Die US 2012/029937 A1 offenbart ein Dialysegerät zur Durchführung einer Peritonealdialyse, das die einstellbare Dauer einer Behandlungssitzung auf einen gewissen Bereich einschränken kann. US 2012/071815 offenbart ein Dialysegerät für die Peritonealdialyse, bei dem aus der Zahl der Zyklen und der Verweildauer pro Zyklus die Behandlungsdauer berechnet werden kann, und die Behandlungsdauer verlängert werden kann, falls das Drainvolumen nicht einen Minimalwert erreicht.

Um eine bessere Planungssicherheit für Betreuer und Patienten (Fahrdienste, Maschinenzeiten etc.) zu erreichen, wäre es wünschenswert, eine zuverlässige Methode zur Hand zu haben, mittels derer eine Abschätzung der Gesamtbehandlungsdauer einer Peritonealdialyse-Behandlung möglich ist. Der vorliegenden Erfindung liegt somit die Aufgabe zugrunde, ein Verfahren zur Abschätzung der voraussichtlichen Behandlungsdauer einer Peritonealdialyse-Behandlung zur Verfügung zu stellen.

Diese Aufgabe wird durch ein Gerät mit den Merkmalen des Anspruchs 1 durchgeführt.

Danach ist vorgesehen, dass die Abschätzung der Behandlungsdauer basierend auf einer idealen Behandlungsdauer vorgenommen wird, wobei die ideale Behandlungsdauer um einen oder mehrere Verzögerungswerte erhöht wird, die von einem oder mehreren patientenspezifischen Parametern abhängen.

Bei diesen Verzögerungswerten kann es sich um absolute Zeitspannen oder auch um relative Zeitspannen handeln, wie beispielsweise um eine prozentuale Erhöhung der Einlaufdauer oder der Auslaufdauer.

Gemäß der Erfindung wird somit zunächst eine ideale Behandlungsdauer bestimmt. Geht man exemplarisch davon aus, dass die Peritonealdialyse-Behandlung einen initialen Auslauf, zwei Zyklen mit jeweils einem Einlauf, einer Verweilzeit in der Bauchhöhle, einem Auslauf und einen letzten Einlauf umfasst, wird die ideale Behandlungsdauer durch die Summation der Dauer des initialen Auslaufes, der Dauer eines Zyklus multipliziert mit der Anzahl der durchgeführten Zyklen sowie der Dauer des letzten Einlaufes ermittelt. Basierend auf diesem Wert werden erfindungsgemäß Zuschläge, d.h. Faktoren, die eine Verlängerung der Behandlungsdauer mit sich bringen, in Form von Verzögerungswerten berücksichtigt, um auf diese Weise zu einer Schätzung der Gesamten Behandlungsdauer zu kommen.

Wie groß die zeitliche Abweichung zwischen der real durchgeführten Behandlung und der Verschreibung ist, hängt im Normalfall von den Eigenschaften des Patienten bzw. von den Eigenschaften des Katheters ab. So ist es denkbar, dass die erwartete Flussrate nicht erreicht wird. Eine weitere Abweichung ergibt sich daraus, dass der Patient Ultrafiltrationsvolumen generiert, so dass sich das erwartete Auslaufvolumen erhöht. Da für druckgesteuerte Ausläufe kein Auslaufvolumen vorgegeben ist, wird für die Berechnung des Auslaufvolumens das sich im Patienten befindliche Volumen angenommen.

Gemäß der Erfindung ist vorgesehen, dass die ideale Behandlungsdauer die ideale Dauer des initialen Auslaufs, die ideale Zyklusdauer multipliziert mit der Anzahl der Zyklen sowie die ideale Dauer des letzten Einlaufes umfasst. Die ideale Dauer des initialen Auslaufes kann bei einem druckgesteuerten Auslauf als ein bestimmter Wert, z.B. 5 min angenommen werden. Bei einem druckgesteuerten Auslauf wird kein bestimmtes Auslaufvolumen vorgegeben, sondern der Patient wird solange entleert, bis kein Volumen mehr aus der Bauchhöhle gefördert werden kann.

Die ideale Zyklusdauer setzt sich vorzugsweise zusammen aus der idealen Einlaufdauer, der idealen Verweilzeit sowie der idealen Auslaufdauer. Die ideale Einlaufdauer kann als Quotient aus dem Einlaufvolumen und der Einlaufrate bestimmt werden. Die Verweilzeit kann vorgegeben werden. Die ideale Auslaufdauer kann aus dem Quotienten aus dem vorgeschriebenen Auslaufvolumen und der Auslaufrate bestimmt werden.

Um dem zeitlich veränderlichen Funktionszustand des Katheters Rechnung zu tragen, ist gemäß der Erfindung vorgesehen, dass ein patientenspezifischer Parameter, der zu einer Verzögerung gegenüber der idealen Behandlungsdauer führen kann, durch die Katherterperformance gebildet wird. Auch wenn die Katherperformance beim Einlauf weniger von dem individuellen Patienten abhängt, wird diese gleichwohl den patientenspezifischen Parametern zugerechnet, da der Katheter im weitesten Sinne Bestandteil des Patienten ist.

Vorzugsweise ist vorgesehen, dass die Katheterperformance bei der Abschätzung der Einlaufdauer und/oder bei der Abschätzung der Auslaufdauer und/oder bei der Abschätzung der Dauer des letzten Einlaufes berücksichtigt wird.

Denkbar ist es, dass die Katherterperformance in Form einer absoluten oder relativen Erhöhung der idealen Einlaufdauer und/oder der idealen Auslaufdauer und/oder der idealen Dauer des letzten Einlaufes berücksichtigt wird. Geht man beispielsweise davon aus, dass die ideale Einlaufdauer 4 min beträgt (Einlaufvolumen: 1000 ml; Einlaufrate: 250 ml/min), kann die Katheterperformance beispielsweise durch den Faktor 110 % berücksichtigt werden, so dass sich als geschätzte Einlaufdauer eine Zeit von 264 s ergibt.

Eine entsprechende Vorgehensweise ist für die Auslaufdauer sowie für die Dauer des letzten Einlaufes denkbar. Wird für die Dauer des initialen Auslaufes keine feste Zeitspanne angenommen, d.h. erfolgt der initiale Auslauf volumengesteuert, ist ebenfalls eine entsprechende Erhöhung der idealen Zeitspannen denkbar.

Die Katheterperformance und insbesondere die absolute oder relative Erhöhung kann editierbar sein, so dass ein Nutzer diese Werte einstellen kann. Dabei ist es denkbar, dass für die unterschiedlichen Phasen unterschiedliche Werte angenommen werden. So kann beispielsweise die relative Verzögerung beim Einlauf 10 % betragen, beim Auslauf jedoch 50 %. Denkbar ist es, dass Default-Werte vorgegeben werden, die dann bei Bedarf nutzerseitig verändert werden können.

Die Einlaufverzögerung aufgrund einer nicht erreichten idealen Einlaufrate ist weniger von Patienten abhängig, so dass vorgesehen sein kann, dass diese nicht am Gerät editierbar ist, d.h. nicht durch einen Nutzer veränderbar ist, sondern aufgrund von Erfahrungswerten fest vorgegeben ist, wie z.B. auf den vorgenannten Wert von 10 %.

Von der Erfindung ist der Fall umfasst, dass nur einige der für die Verzögerung relevanten Parameter nutzerseitig veränderbar sind oder dass alle oder keiner dieser Parameter durch den Nutzer veränderbar ist. Im letzten Fall sind die Verzögerungswerte sämtlich maschinenseitig fest eingestellt.

Wie oben ausgeführt, kann vorgesehen sein, dass der initiale Auslauf druckgesteuert erfolgt und dass die Dauer des initialen Auslaufes geräteseitig vorgegeben ist oder durch einen Nutzer eingestellt werden kann. So kann beispielsweise ein Wert von 5 min für den initialen Auslauf angesetzt werden.

Von der Erfindung ist auch der Fall umfasst, dass der initiale Auslauf volumengesteuert erfolgt und dass die Dauer des initialen Auslaufes aus einem (geschätzten) initialen Auslaufvolumen und einer Auslaufrate bestimmt wird. Auch die Dauer des initialen Auslaufes kann zur Berücksichtigung der Katheterperformance um einen absoluten oder relativen Verzögerungswert erhöht werden.

Bei dem verfahrensspezifischen Parameter kann es sich beispielsweise um eine absolute oder relative Verzögerung beim Einlauf und/oder um eine Verzögerung beim Auslauf handeln. Auch diese Werte können fest vorgegeben sein oder durch einen Nutzer veränderbar sein. Denkbar ist es beispielsweise, dass pro Auslauf eine Verzögerung von 1 min und pro Einlauf eine Verzögerung von 2 min angesetzt wird. Diese verfahrensbedingten Verzögerungen hängen somit nicht vom geförderten Volumen ab.

Wird beispielsweise als ideale Einlaufdauer eine Zeitspanne von 4 min angenommen, kann die Katheterperfomance um einen Zuschlag um z.B. 10 % erhöht werden. Geht man von einer verfahrensbedingten Verzögerung von z.B. 120 s aus, ergibt sich eine Einlaufdauer von 384 s (= 240 s * 110% + 120 s).

Eine Unbekannte ist das Volumen für den initialen Auslauf, wenn dieser als druckgesteuerter Auslauf verschrieben wurde. Dabei wird der Patient solange entleert, bis keine Volumen mehr durch den Katheter in die Drainage gefördert wird. Nimmt man für den initialen Auslauf eine bestimmte Dauer, wie z.B. 5 min an, kann auf der Basis dieser Annahme bei Kenntnis der Förderrate für den Auslauf ein theoretisches Volumen berechnet werden, das während dieser bestimmten Dauer gefördert werden kann. Das Volumen des initialen Auslaufes kann somit aus der effektiven Dauer des initialen Auslaufs und aus der effektiven Auslaufrate ermittelt werden.

Dabei kann die Dauer des initialen Auslaufes aus einer vorgegebenen Zeit zuzüglich einer verfahrensmäßigen Verzögerung, z.B. in der Länge von 1 min, bestimmt werden.

Weiterhin kann vorgesehen sein, dass die effektive Auslaufrate aus der vorgegebenen Auslaufrate und einem die Katherterperformance berücksichtigenden Wert bestimmt wird, der z.B. in Form eines prozentualen Zuschlages erfasst wird.

Das auf diese Weise bestimmte Volumen des initialen Auslaufes kann herangezogen werden, um eine Grafik zu erstellen, die das jeweils im Patienten befindliche Volumen der Dialyseflüssigkeit über die Zeit zeigt.

Weiterhin kann vorgesehen sein, dass für die geschätzte Dauer der Verweilzeit der Lösung im Patienten und/oder für die geschätzte Dauer einer Behandlungspause die vorgeschriebene Dauer verwendet wird. Wird beispielsweise für die Verweilzeit ein Wert von 60 min vorgeschrieben, kann dieser Wert auch für die Schätzung herangezogen werden, da nicht davon auszugehen ist, dass eine Verlängerung der Verweilzeit eintritt. Entsprechendes gilt für Behandlungspausen.

Für die geschätzte Dauer einer Benachrichtigung kann der Wert Null angenommen werden, da eine Benachrichtigung keinen Niederschlag in der Behandlungsdauer findet.

Gemäß einer bevorzugten Ausführungsform der Erfindung setzt sich die geschätzte Behandlungsdauer aus den folgenden geschätzten Zeiten zusammen, wobei die angegebenen Dauern bzw. Phasen je nach Behandlungsverlauf gar nicht, genau einmal oder auch mehrfach vorkommen können: initiale Auslaufdauer, Einlaufdauer, Auslaufdauer, Verweildauer im Patienten, finale Auslaufdauer, Dauer der Behandlungspause, Dauer von Benachrichtigungen, wobei für letztere Vorzugsweise der Wert Null angenommen wird und wobei für die Verweildauer im Patienten und für die Dauer der Behandlungspause vorzugsweise die vorgeschriebenen, d.h. die bekannten Werte verwendet werden.

In weiterer Ausgestaltung der Erfindung ist vorgesehen, dass effektive Einlaufraten und/oder Auslaufraten ermittelt werden und dass zum Zwecke der Erstellung einer Trendanalyse durch eine Aufzeichnung dieser Daten über die Zeit eine Änderung der Katheterperformance ermittelt wird. Wird das Volumen der Behandlungsflüssigkeit im Patienten über die Zeit aufgetragen, so repräsentiert die Steigung des Graphen die Einlaufrate bzw. die Auslaufrate. Findet eine Aufzeichnung über einen längeren Zeitraum statt, können Aussagen darüber getroffen werden, ob sich die Raten und damit die Katheterperformance geändert hat.

Um für folgende Behandlungen möglichst realistische Schätzwerte abgeben zu können, kann vorgesehen sein, dass die geänderte Katheterperformance der Abschätzung der Behandlungsdauer zukünftiger Behandlungen zugrunde gelegt wird. So ist es beispielsweise denkbar, für die Auslaufverzögerung statt des Faktors 150 % nunmehr 160 % anzusetzen, wenn sich gezeigt hat, dass die Katheterperformance entsprechend nachgelassen hat.

Denkbar ist es weiterhin, dass die abgeschätzte Behandlungsdauer mit einem oberen und/oder mit einem unteren Grenzwert verglichen wird und dass ein Hinweis an den Nutzer ausgegeben wird, wenn die abgeschätzte Behandlungsdauer den oberen Grenzwert übersteigt oder den unteren Grenzwert unterschreitet. Auf diese Weise kann festgestellt werden, ob die geplante Behandlung unter realistischen Vorgaben durchgeführt werden soll oder ob die Korrektur eines oder mehrerer Parameter notwendig ist.

In einer weiteren Ausgestaltung der Erfindung ist vorgesehen, dass die Verweilzeit der Behandlung in abgeschätzter Behandlungsdauer geändert wird oder unverändert bleibt. Im Rahmen der Durchführung von Behandlungen können somit Verweilzeiten, d.h. die Zeitspannen, in denen sich die Dialysierflüssigkeit in der Bauchhöhle befindet, ggf. verkürzt werden. Ist beispielsweise als gesamte Behandlungsdauer eine bestimmte Zeitdauer vorgegeben und liegt die abgeschätzte Behandlungsdauer über dieser vorgegebenen Zeitdauer, kann vorgesehen sein, die Verweilzeit(en) entsprechend so zu kürzen, dass die vorgegebene Zeitdauer eingehalten wird, dass also die Schätzung mit korrigierten Verweilzeiten genau oder weitgehend der vorgegebenen Zeitdauer entspricht, was auch mit einschließt, dass die Schätzung in einem zulässigen Bereich liegt.

Liegt die geschätzte Dauer hingegen in einem zulässigen Bereich oder entspricht dieses einem zulässigen Wert bedarf es keiner Änderung der Verweilzeiten und die Behandlung kann wie geplant durchgeführt werden.

Die vorliegende Erfindung betrifft ein Dialysegerät zur Durchführung einer Peritonealdialyse mit wenigstens einer Steuer- oder Regelungseinheit, mit wenigstens einer Eingabeeinheit zur Eingabe eines oder mehrerer Werte, sowie mit wenigstens einem Prozessor, wobei der Prozessor programmiert ist, um ein Verfahren gemäß einem der vorstehend dargestellten Varianten durchzuführen. Des Weiteren weist das Dialysegerät vorzugsweise einen oder mehrere Speicher auf, um z.B. Grenzwerte zulässiger Bereiche zu speichern oder um vorgegebene Werte, z.B. einen geräteseitig vorgegebenen Wert für eine Verzögerung z.B. im Rahmen des Einlaufes etc. zu speichern. Der Speicher kann auch dazu dienen, die nutzerseitig eingegebenen Daten zu speichern.

In einer bevorzugten Ausgestaltung der Erfindung ist vorgesehen, dass das Dialysegerät mit wenigstens einer Einrichtung versehen ist, die derart ausgebildet ist, dass sie einen zeitlichen Verlauf des Volumens der Dialyselösung im Patienten ermittelt. Dieser zeitliche Verlauf kann dann beispielsweise ausgedruckt oder für einen Nutzer sichtbar angezeigt werden.

Auch ist es denkbar, dass das Gerät Mittel aufweist, die derart ausgebildet sind, dass diese die Steigung der Volumenzunahme (beim Einlauf) und/oder der Volumenabnahme (beim Auslauf) ermittelt und mehrere zeitlich voneinander beabstandete Steigungen miteinander vergleicht. Aus diesem Vergleich kann dann ermittelt werden, ob eine Änderung der Steigung eingetreten ist, was auf eine Änderung der Katheterperformance zurückzuführen ist. Sofern dies der Fall ist, kann geräteseitig eine Änderung von Default-Werten erfolgen oder es kann dem Nutzer ein Vorschlag für einen Wert für die Katheterperformance vorgeschlagen werden.

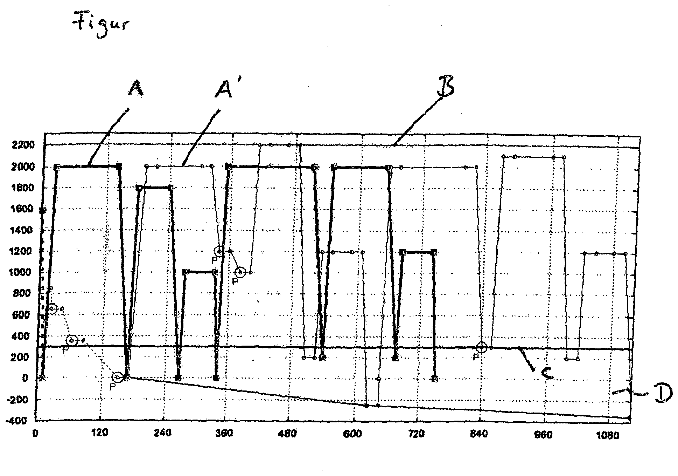

Weitere Einzelheiten und Vorteile der Erfindung werden anhand eines in der Zeichnung dargestellten Ausführungsbeispiels näher erläutert. Die einzige Figur zeigt einen zeitlichen Verlauf des einem Patienten verabreichten Volumens einer Dialyselösung gemäß einer Schätzung sowie den tatsächlichen zeitlichen Verlauf des dem Patienten verabreichten Volumens der Dialyselösung.

Gemäß dem in Folgenden beschrieben Beispiel ist eine Standardverschreibung mit den folgenden Parametern gegeben:

- Durchführung von zwei Zyklen
- Druckgesteuerter initialer Auslauf
- Verweilzeit mit je 60 min
- Einlauf mit je 1000 ml
- Letzter Einlauf mit 1250 ml

- Einlaufrate mit 250 ml/min
- Auslaufrate mit 200 ml/min

Die Berechnung der idealen Behandlungszeit, d.h. der Behandlungszeit, bei der weder verfahrensbedingte noch patientenbedingte oder sonstige Verzögerungen eintreten, gestaltet sich wie folgt:
Die initiale ideale Auslaufdauer wird mit 5 min angenommen:

$$\Delta t_{Auslauf,initial,\ ideal} = 5\ min$$

Dieser Wert kann einstellbar sein oder geräteseitig vorgegeben werden.

Die ideale Einlaufdauer ergibt sich aus dem Quotienten des dem Patienten zu verabreichenden Volumens mit der vorgeschriebenen bzw. eingestellten Einlaufrate:

$$\Delta t_{Einlauf,\ ideal} = V_{Einlauf}/Q_{Einlauf} = 1000\ ml/250\ ml/min = 4\ min.$$

Die ideale Auslaufdauer ergibt sich aus dem Quotienten des abzuführenden Volumens der Dialyselösung mit der vorgeschriebenen bzw. eingestellten Auslaufrate. Geht man davon aus, dass das abzuführende Volumen gleich dem verabreichten Volumen ist, ergibt sich:

$$\Delta t_{Auslauf,\ ideal} = V_{Auslauf}/Q_{Auslauf} = 1000\ ml/200\ ml/min = 5\ min.$$

Ein Zyklus setzt sich zusammen aus einem Einlauf, einer Verweilperiode sowie einem Auslauf. Die ideale Zyklusdauer $\Delta t_{Zyklus,\ ideal}$ beträgt:

$$\Delta t_{Zyklus,\ ideal} = \Delta t_{Einlauf,\ ideal} + \Delta t_{Verweildauer,\ ideal} + \Delta t_{Auslauf,\ ideal} = 4\ min + 60\ min + 5\ min = 69\ min.$$

Die ideale Dauer des letzten Einlaufs ergibt sich aus dem Quotienten aus dem Einlaufvolumen (1250 ml) und der Einlaufrate (250 ml/min):

$$\Delta t_{Einlauf,\ final,\ ideal} = V_{Einlauf}/Q_{Einlauf} = 1250\ ml/250\ ml/min = 5\ min.$$

Daraus ergibt sich die gesamte Behandlungsdauer $\Delta t_{Gesamt,\ ideal}$ wie folgt:

$$\Delta t_{Gesamt,\ ideal} = \Delta t_{Auslauf,initial,\ ideal} + 2 * \Delta t_{Zyklus,\ ideal} + \Delta t_{Einlauf,\ final,\ ideal} =$$

$$5\ min + 2 * 69\ min + 5\ min = 148\ min.$$

Die ideale Gesamtbehandlungsdauer für dieses Beispiel beträgt somit 148 min. Diese Behandlungsdauer ergibt sich, wenn es keine verfahrensbedingten Verzögerungen, wie Pausen oder Verzögerungen beim Ein- oder Auslauf, und keine nicht verfahrensbedingten Verzögerungen, wie patienten- oder durch das verwendete Equipment bedingte Verzögerungen, gibt.

Legt man nun zu erwartende verfahrensbedingte sowie patientenbedingte Verzögerungen zu Grunde, ergibt sich für denselben Behandlungsverlauf (2 Zyklen) eine geschätzte Gesamtbehandlungsdauer wie folgt:

$$\Delta t_{Gesamt,\ geschätzt} = \Delta t_{Auslauf,initial} + 2 * \Delta t_{Zyklus} + \Delta t_{Einlauf,\ final}$$

Für die Dauer des initialen Auslaufs wird wieder eine Zeitspanne von 5 min angenommen.

Die Zyklusdauer $\Delta t_{Zyklus}$ ergibt sich wieder aus $\Delta t_{Einlauf} + \Delta t_{Verweildauer} + \Delta t_{Auslauf}$.

Für den Einlauf wird eine verfahrensbedingte Verzögerung $\Delta t_{Einlauf}$, Verzögerung von 120 s angenommen. Für den Einlauf wird des Weiteren eine Verzögerung durch eine verringerte Katheterperformance betreffend den Einlauf um den Faktor 110 % angenommen, d.h. die Einlaufdauer ist bedingt durch eine Eigenschaft des Katheters um 10 % größer, als im Idealzustand, in dem diese Eigenschaft nicht vorliegt. Benennt man den die Verzögerung beim Einlauf betreffenden Katheterparameter $F_{\Delta tEinlauf}$, ergibt sich für die Einlaufdauer:

$$\Delta t_{Einlauf} = V_{Einlauf}/Q_{Einlauf} * F_{\Delta tEinlauf} + \Delta t_{Einlauf, Verzögerung} = 1000\ ml/250\ ml/min *$$
$$110/100 + 120\ s = 264\ s + 120\ s = 384\ s = 6\ min\ 24\ s.$$

Der Wert für $F_{\Delta tEinlauf}$ kann geräteseitig mit 110 % voreingestellt sein. Grundsätzlich ist jedoch auch denkbar, dass der Wert durch einen Nutzer einstellbar ist.

Für die Auslaufdauer $\Delta t_{Auslauf}$ ergibt sich bei einer Verzögerung bedingt durch den Katheter beim Auslauf $F_{\Delta tAuslauf}$ und bei einer verfahrensbedingten Verzögerung $\Delta t_{Auslauf}$, Verzögerung von 60 s Folgendes:

$$\Delta t_{Auslauf} = V_{Auslauf}/Q_{Auslauf} * F_{\Delta tAuslauf} + \Delta t_{Auslauf, Verzögerung} = 1000\ ml/200\ ml/min *$$
$$150/100 + 60\ s = 510\ s = 8\ min\ 30\ s.$$

Nimmt man für die Dauer des letzten Einlaufes erneut einen Wert $F_{\Delta tEinlauf}$ in Höhe von 110% und eine Verzögerung von 120 s an, ergibt sich

$$\Delta t_{Einlauf, final} = V_{Einlauf}/Q_{Einlauf} * F_{\Delta tEinlauf} = 1250\ ml/250\ ml/min * 110/100 + 120\ s = 450$$
$$s = 7\ min.$$

Die Werte $\Delta t_{Einlauf}$, Verzögerung und $\Delta t_{Auslauf}$, Verzögerung können ihre Ursache in verfahrensbedingten Verzögerungen, wie beispielsweise in dem Einmessen des Patientendruckes, in einem Beutelwechsel während des Einlaufes etc. haben.

Die Werte $F_{\Delta tEinlauf}$ und $F_{\Delta tAuslauf}$ können beispielsweise zwischen einem Minimalwert (100 %) und einem Maximalwert (200 %) schwanken. Für beide Werte kann ein Defaultwert, beispielsweise 130 % vorgegeben bzw. vorgeschlagen werden.

Die Zyklusdauer bei einer angenommenen Verweilzeit von 60 min beträgt nunmehr somit:

$$\Delta t_{Zyklus} = 384\ s + 3600\ s + 510\ s = 4494\ s = 74\ min\ 54\ s.$$

Bei zwei Zyklen erhält man dann die gesamte Behandlungsdauer nach:

$$\Delta t_{Gesamt, geschätzt} = \Delta t_{Auslauf,initial} + 2 * \Delta t_{Zyklus} + \Delta t_{Einlauf, final} = 300\ s + 2 * 4494\ s + 450\ s$$
$$= 9708\ s = 161\ min\ 48\ s \approx 162\ min.$$

Geht man davon aus, dass der Wert für $F_{\Delta tAuslauf}$ editierbar, d.h. nutzerseitig veränderbar ist, ergibt sich die folgende Tabelle für die Auslaufdauer und für die geschätzte Gesamtdauer der Behandlung für unterschiedliche Werte für $F_{\Delta tAuslauf}$:

| $F_{\Delta tAuslauf}$ [%] | $\Delta t_{Einlauf}$ [s] | $\Delta t_{Gesamt, geschätzt}$ [min] |
|---|---|---|
| 100 | 360 | 157 |
| 110 | 390 | 158 |
| 120 | 420 | 159 |

(fortgesetzt)

| $F_{\Delta tAuslauf}$ [%] | $\Delta t_{Einlauf}$ [s] | $\Delta t_{Gesamt, geschätzt}$ [min] |
|---|---|---|
| 130 | 450 | 160 |
| 140 | 480 | 161 |
| 150 | 510 | 162 |
| 160 | 540 | 163 |
| 170 | 570 | 164 |
| 180 | 600 | 165 |
| 190 | 630 | 166 |
| 200 | 660 | 167 |

Geht man davon aus, dass des Weiteren Zeitspannen für eine oder mehrere Pausen $\Delta t_{Pause}$ und/oder Nachrichten $\Delta t_{Nachricht}$ zu berücksichtigen sind, verlängert sich die geschätzte Zyklusdauer sowie die geschätzte Gesamtdauer entsprechend. Dabei kann die geschätzte Dauer der Pause der vorgeschriebenen Dauer der Pause entsprechen. Entsprechendes gilt für die Verweildauer der Dialyselösung im Patienten. Für die Dauer einer Nachricht kann der Wert Null angenommen werden.

Wie oben ausgeführt, kann bei einem druckgesteuerten initialen Auslauf das initiale Auslaufvolumen $V_{Auslauf,initial}$ abgeschätzt werden. Die Abschätzung kann unter Zuhilfenahme der Beziehung

$$V_{Auslauf,initial} = \Delta t_{Auslauf,initial,\, eff} * Q_{Auslauf,initial,\, eff}$$

erfolgen. Die effektive Auslaufrate $Q_{Auslauf,initial,eff}$ ergibt sich aus der vorgeschriebenen Auslaufrate $Q_{Auslauf,initial,vorgeschrieben}$ gemäß:

$$Q_{Auslauf,initial,\, eff} = Q_{Auslauf,initial,vorgeschrieben} / F_{\Delta tAuslauf}.$$

Die effektive Auslaufdauer $\Delta t_{Auslauf,initial,\, eff}$ ergibt sich aus

$$\Delta t_{Auslauf,initial,\, eff} = \Delta t_{Auslauf,initial,\, vorgeschrieben} + \Delta t_{Auslauf,\, Verzögerung}.$$

Für $F_{\Delta tAuslauf}$ und für $\Delta t_{Auslauf,initial,}$ vorgeschrieben können beispielsweise die oben genannten Werte von 110 % und 5 min eingesetzt werden.

Nach dieser Beziehung kann das initiale Auslaufvolumen $V_{Auslauf,initial}$ ermittelt werden.

Die Figur zeigt gemäß Verlauf A den mittels des erfindungsgemäßen Verfahrens geschätzten Verlauf und der Verlauf A' den tatsächlichen Verlauf des in dem Patienten befindlichen Volumens der Dialyselösung über die Zeit. Die Markierungen P symbolisieren Druckereignisse.

Wie dies aus der Figur ersichtlich ist, beginnt der Verlauf A mit einer gepunkteten Linie, die mit dem berechneten anfänglichen Auslaufvolumen beginnt und sich über eine festgelegte Zeitspanne von 5 min erstreckt.

Nach einer Einlaufphase beginnt die Verweilzeit im Patienten, der eine Auslaufphase auf das Volumen Null folgt. Anschließend wiederholen sich Einlaufphasen, Verweilphasen und Auslaufphasen, wie dies aus der Figur hervorgeht.

Die Linie B kennzeichnet das maximal zulässige Volumen der Dialyselösung im Patienten, die Linie C kennzeichnet das maximal zulässige im Patienten verbleibende Volumen.

Der Bereich D kennzeichnet das während der Behandlung entzogene Ultrafiltrationsvolumen. Die initiale Auslaufphase wird bei der Bestimmung des Ultrafiltrationsvolumens nicht berücksichtigt.

Eine Trendanalyse über längere Zeiträume könnte eine Aussage darüber treffen, ob und wie sich die Eigenschaften des Katheters über die Zeit verändern. Die effektiven Flussraten beim Auslauf und Einlauf entsprechen den Steigungen des Verlaufs A'. Ist die Steigung Null findet weder ein Auslauf noch ein Einlauf statt. Diese ist beispielsweise während der Verweilphasen oder bei Behandlungspausen oder verfahrensbedingten Stillstandzeiten der Fall.

Vorzugsweise wird jede Behandlung protokolliert. Das Protokoll kann die mittlere effektive Flussrate für Einlauf und Auslauf, d.h. die mittlere effektive Einlauf- und Auslaufrate umfassen, die über die gesamte Behandlung berechnet werden.

Die berechneten Werte berücksichtigen nur die Flussraten, in denen effektiv Volumen in den bzw. aus dem Körper gefördert wurde, d.h. die Zeiten, in denen sich die Pumpen bewegten. Die effektiven Flussraten können darüber hinaus für alle Phasen protokolliert werden. Wie oben ausgeführt, lässt sich durch einen Vergleich verschiedener effektiven Flussraten zu unterschiedlichen Zeitpunkten ein Rückschluss auf die Katheterperformance ziehen.

**Patentansprüche**

1. Dialysegerät zum Durchführen einer Peritonealdialyse mit einer Steuer- oder Regelungseinheit, einer Eingabeeinheit zur Eingabe eines oder mehrerer Werte und einem Prozessor, der dazu programmiert ist, eine Abschätzung einer Behandlungsdauer einer Peritonealdialyse-Behandlung durchzuführen, wobei hierbei

   die Abschätzung der Behandlungsdauer basierend auf einer idealen Behandlungsdauer vorgenommen wird, die ideale Behandlungsdauer um einen oder mehrere Verzögerungswerte erhöht wird, wobei die ideale Behandlungsdauer die ideale Dauer des initialen Auslaufs, die ideale Zyklusdauer multipliziert mit der Anzahl der Zyklen sowie die ideale Dauer des letzten Einlaufes umfasst und
   die Verzögerungswerte von einem oder mehreren patientenspezifischen Parametern abhängen, wobei ein patientenspezifischer Parameter durch die Katheterperformance gebildet wird.

2. Gerät nach Anspruch 1, **dadurch gekennzeichnet, dass** die Katheterperformance bei der Abschätzung der Einlaufdauer und/oder bei der Abschätzung der Auslaufdauer und/oder bei der Abschätzung der Dauer des letzten Einlaufes berücksichtigt wird, wobei vorzugsweise vorgesehen ist, dass die Katheterperformance in Form einer absoluten oder relativen Erhöhung der idealen Einlaufdauer und/oder der idealen Auslaufdauer und/oder der idealen Dauer des letzten Einlaufes berücksichtigt wird.

3. Gerät nach Anspruch 2, **dadurch gekennzeichnet, dass** die die absolute oder relative Erhöhung der Katheterperformance durch einen Nutzer einstellbar ist.

4. Gerät nach einem der vorhergehenden Ansprüche, **dadurch gekennzeichnet, dass** der initiale Auslauf druckgesteuert erfolgt und dass die Dauer des initialen Auslaufes geräteseitig vorgegeben ist, oder dass der initiale Auslauf volumengesteuert erfolgt und dass die Dauer des initialen Auslaufes aus einem initialen Auslaufvolumen und einer Auslaufrate bestimmt wird, wobei vorzugsweise vorgesehen ist, dass zur Berücksichtigung der Katheterperformance eine Erhöhung des auf diese Weise bestimmten Wertes erfolgt.

5. Gerät nach einem der vorhergehenden Ansprüche, **dadurch gekennzeichnet, dass** das Volumen des initialen Auslaufes aus der effektiven Dauer des initialen Auslaufs und aus der effektiven Auslaufrate ermittelt wird.

6. Gerät nach Anspruch 5, **dadurch gekennzeichnet, dass** die effektive Dauer eine vorgegebene Dauer zuzüglich einer Zeitverzögerung ist und/oder dass die effektive Auslaufrate aus der vorgegebenen Auslaufrate und einem die Katheterperformance berücksichtigenden Wert bestimmt wird.

7. Gerät nach einem der vorhergehenden Ansprüche, **dadurch gekennzeichnet, dass** für die geschätzte Dauer der Verweilzeit der Lösung im Patienten und/oder für die geschätzte Dauer einer Behandlungspause die vorgeschriebene Dauer verwendet wird und/oder dass für die geschätzte Dauer einer Benachrichtigung der Wert Null angenommen wird.

8. Gerät nach einem der vorhergehenden Ansprüche, **dadurch gekennzeichnet, dass** sich die geschätzte Behandlungsdauer aus der oder den folgenden geschätzten Zeiten zusammensetzt: initiale Auslaufdauer, Einlaufdauer, Auslaufdauer, Verweildauer im Patienten, Dauer der Behandlungspause, Dauer von Benachrichtigungen, finale Einlaufdauer.

9. Gerät nach einem der vorhergehenden Ansprüche, **dadurch gekennzeichnet, dass** die effektiven Einlaufraten und/oder Auslaufraten ermittelt werden und dass zum Zwecke der Erstellung einer Trendanalyse durch eine Aufzeichnung dieser Daten über die Zeit eine Änderung der Katheterperformance ermittelt wird, wobei vorzugsweise vorgesehen ist, dass die geänderte Katheterperformance der Abschätzung der Behandlungsdauer zukünftiger Behandlungen zugrunde gelegt wird.

10. Gerät nach einem der vorhergehenden Ansprüche, **dadurch gekennzeichnet, dass** die Verweilzeit der Behandlung in Abhängigkeit der abgeschätzten Behandlungsdauer geändert wird oder unverändert bleibt.

11. Gerät nach einem der vorhergehenden Ansprüche, **dadurch gekennzeichnet, dass** die abgeschätzte Behandlungsdauer mit einem oberen und/oder mit einem unteren Grenzwert verglichen wird und dass ein Hinweis an den Nutzer ausgegeben wird, wenn die abgeschätzte Behandlungsdauer den oberen Grenzwert übersteigt oder den unteren Grenzwert unterschreitet.

**Claims**

1. Dialysis device for performing a peritoneal dialysis, comprising an open- or closed-loop control unit, an input unit for entering one or more values, and a processor, which is programmed to perform an estimation of a treatment duration of a peritoneal dialysis treatment, wherein

   the estimation of the treatment duration is carried out on the basis of an ideal treatment duration, the ideal treatment duration is increased by one or more delay values, wherein the ideal treatment duration comprises the ideal duration of the initial outflow, the ideal cycle duration multiplied by the number of cycles and the ideal duration of the last inflow, and the delay values depend on one or more patient-specific parameters, wherein a patient-specific parameter is formed by the catheter performance.

2. Device according to claim 1, **characterised in that** the catheter performance is taken into account during the estimation of the inflow duration and/or during the estimation of the outflow duration and/or during the estimation of the duration of the last inflow, wherein preferably it is provided that the catheter performance is taken into account in the form of an absolute or relative increase in the ideal inflow duration and/or the ideal outflow duration and/or the ideal duration of the last inflow.

3. Device according to claim 2, **characterised in that** the absolute or relative increase in the catheter performance can be set by a user.

4. Device according to one of the preceding claims, **characterised in that** the initial outflow takes place in a pressure-controlled manner and that the duration of the initial outflow is preset by the device, or that the initial outflow takes place in a volume-controlled manner and that the duration of the initial outflow is determined from an initial outflow volume and an outflow rate, wherein preferably it is provided that the value determined in this manner is increased in order to take the catheter performance into account.

5. Device according to one of the preceding claims, **characterised in that** the volume of the initial outflow is determined from the effective duration of the initial outflow and from the effective outflow rate.

6. Device according to claim 5, **characterised in that** the effective duration is a preset duration in addition to a time delay and/or that the effective outflow rate is determined from the preset outflow rate and a value taking the catheter performance into account.

7. Device according to one of the preceding claims, **characterised in that** for the estimated duration of the dwell time of the solution in the patient and/or for the estimated duration of a treatment break the specified duration is used and/or that for the estimated duration of a notification the value zero is assumed.

8. Device according to one of the preceding claims, **characterised in that** the estimated treatment duration is composed of the following estimated time(s): initial outflow duration, inflow duration, outflow duration, dwell time in the patient, duration of the treatment break, duration of notifications, final inflow duration.

9. Device according to one of the preceding claims, **characterised in that** the effective inflow rates and/or outflow rates are determined and that, for the purpose of establishing a trend analysis by recording this data over time, a change in catheter performance is determined, wherein preferably it is provided that the changed catheter performance is used as a basis for estimating the treatment duration of future treatments.

10. Device according to one of the preceding claims, **characterised in that** the dwell time of the treatment is changed depending on the estimated treatment duration or remains unchanged.

11. Device according to one of the preceding claims, **characterised in that** the estimated treatment duration is compared to an upper and/or lower threshold value and that an indication is given to the user if the estimated treatment duration exceeds the upper threshold value or falls below the lower threshold value.

**Revendications**

1. Appareil de dialyse pour réaliser une dialyse péritonéale avec une unité de commande ou de régulation, une unité de saisie pour saisir une ou plusieurs valeurs et un processeur qui est programmé pour réaliser une estimation d'une durée de traitement d'un traitement de dialyse péritonéale, dans lequel dans le cas présent

   l'estimation de la durée de traitement est effectuée sur la base d'une durée de traitement idéale,
   la durée de traitement idéale est augmentée d'une ou de plusieurs valeurs de temporisation, dans lequel la durée de traitement idéale comprend la durée idéale de la sortie initiale, la durée de cycle idéale multipliée par le nombre de cycles et la durée idéale de la dernière entrée, et
   les valeurs de temporisation dépendent d'un ou de plusieurs paramètres propres au patient, dans lequel un paramètre propre au patient est formé par la performance de cathéter.

2. Appareil selon la revendication 1, **caractérisé en ce que** la performance de cathéter est prise en compte lors de l'estimation de la durée d'entrée et/ou lors de l'estimation de la durée de sortie et/ou lors de l'estimation de la durée de la dernière entrée, dans lequel il est prévu de préférence que la performance de cathéter est prise en compte sous la forme d'une augmentation absolue ou relative de la durée d'entrée idéale et/ou de la durée de sortie idéale et/ou de la durée idéale de la dernière entrée.

3. Appareil selon la revendication 2, **caractérisé en ce que** l'augmentation absolue ou relative de la performance de cathéter peut être réglée par un utilisateur.

4. Appareil selon l'une quelconque des revendications précédentes, **caractérisé en ce que** la sortie initiale se fait avec une commande de pression, et que la durée de la sortie initiale est spécifiée du côté de l'appareil, ou que la sortie initiale se fait avec une commande de volume et que la durée de la sortie initiale est définie à partir d'un volume de sortie initial et d'une vitesse de sortie, dans lequel il est prévu de préférence qu'une augmentation de la valeur ainsi définie se fait pour prendre en compte la performance de cathéter.

5. Appareil selon l'une quelconque des revendications précédentes, **caractérisé en ce que** le volume de la sortie initiale est déterminé à partir de la durée réelle de la sortie initiale et à partir de la vitesse de sortie réelle.

6. Appareil selon la revendication 5, **caractérisé en ce que** la durée réelle est une durée spécifiée plus un temps de temporisation, et/ou que la vitesse de sortie réelle est définie à partir de la vitesse de sortie spécifiée et d'une valeur prenant en compte la performance de cathéter.

7. Appareil selon l'une quelconque des revendications précédentes, **caractérisé en ce que** la durée prescrite est utilisée pour la durée estimée du temps de séjour de la solution dans le patient et/ou pour la durée estimée d'une pause de traitement, et/ou que la valeur de zéro est adoptée pour la durée estimée d'une notification.

8. Appareil selon l'une quelconque des revendications précédentes, **caractérisé en ce que** la durée de traitement estimée se compose du ou des temps estimés qui suivent : durée de sortie initiale, durée d'entrée, durée de sortie, durée de séjour dans le patient, durée de la pause de traitement, durée de notifications, durée d'entrée finale.

9. Appareil selon l'une quelconque des revendications précédentes, **caractérisé en ce que** les vitesses d'entrée et/ou vitesses de sortie réelles sont déterminées, et qu'une modification de la performance de cathéter est déterminée par un enregistrement desdites données sur le temps aux fins de la création d'une analyse de tendance, dans lequel il est prévu de préférence que la performance de cathéter modifiée se fonde sur l'estimation de la durée de traitement de futurs traitements.

10. Appareil selon l'une quelconque des revendications précédentes, **caractérisé en ce que** la durée de séjour du traitement est modifiée en fonction de la durée de traitement estimée ou reste inchangée.

11. Appareil selon l'une quelconque des revendications précédentes, **caractérisé en ce que** la durée de traitement estimée est comparée à une valeur limite supérieure et/ou à une valeur limite inférieure, et qu'un message est envoyé à l'utilisateur quand la durée de traitement estimée dépasse la valeur limite supérieure ou descend sous la valeur limite inférieure.

Figur

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2012029937 A1

- US 2012071815 A